# EUROPEAN PATENT APPLICATION

(11) **EP 1 253 509 A1**
(43) Date of publication of application: **30.10.2002**
(21) Application number: 01870092.2
(22) Date of filing: 27.04.2001
(51) Int. Cl.: G06F 3/00, G06K 11/18, A61B 1/005

(54) **Device for controlling a three-dimensional movement**

(71) Applicant: Andre, Jacques, 1410 Waterloo (BE); Polet, Roland, 6900 Marche-en-Famennes (BE)
(72) Inventor: Andre, Jacques, 1410 Waterloo (BE); Polet, Roland, 6900 Marche-en-Famennes (BE)
(74) Representative: Van Malderen, Joelle

(57) **Abstract**

The present invention is related to a device for translating spatial movements of a finger of the user of said device, into spatial movements of an object in wired or wireless connection with said device, said device comprising a command part (3) which is connected by electrical connections (4) to a transmission part (2), characterised in that said command part is in physical contact only with the first phalanx (200) of said finger, during operation of said device.

## Description

### Field of the invention

The present invention is related to a device for controlling a three-dimensional movement, such as a mouse or joystick or pedal.

### State of the art

Devices such as a mouse or a joystick are generally able to command a movement in two dimensions, meaning in the direction of two orthogonal axes, X and Y. Some types of joysticks provide the additional control of the movement along a third (Z) axis, perpendicular to the other two. These latter devices normally have two buttons, each one commanding the movement in one or the other direction along said third axis. These buttons, which can be placed next to a joystick make it necessary for the user operating the device, to remove his hand or foot or at least one finger from the device, in order to select the direction to be used. In other cases, several members, e.g. several fingers need to be used in order to combine the control of the two-dimensional movement with that of the Z-movement.

Document JP11154031 describes a joy stick capable of inputting components of three systems at a time with one finger. This is done by an operation ring into which a finger or arm is inserted. In the common case, this member is the thumb and the ring is placed on top of the joystick. Moving the thumb up- or downwards is then equal to selecting the movement in either direction along the third axis. This design mainly has a security problem in some applications, in particular medical applications (command of laprascopic equipment). The main problem is the fact that the finger or arm controlling the sixth movement cannot easily or quietly be removed from the ring. Also, the movement of the thumb in an upward direction is not easily maintained for long periods of time, i.e. this movement is not ergonomic and will lead to errors which are unacceptable in medical applications.

### Aims of the invention

The present invention aims to provide a device for controlling a three-dimensional movement, having none of the disadvantages of the prior art.

### Summary of the invention

The present invention is related to a device for translating spatial movements of a finger of the user of said device, into spatial movements of an object in wired or wireless connection with said device, said device comprising a command part which is connected by electrical connections to a transmission part, characterised in that said command part is in physical contact only with the first phalanx of said finger, during operation of said device.

Preferably, said movements take place in a maximum of six directions (+X, -X, +Y, -Y, +Z, -Z), each direction being parallel to one of three orthogonal axes (X, Y, Z) .

According to a preferred embodiment, said command part is strapped around said phalanx, and held by said phalanx against an essentially rigid surface, during operation of said device.

According to the first embodiment of a device according to the invention, said command part comprises :
- a semi-rigid part that fits on the user's finger's first phalanx,
- a flexible part enveloping both said semi-rigid part and said phalanx and making contact with said surface,
- a first contact,
- an electrically conducting lever, in permanent contact with said first contact,
- a cylinder-like enclosure placed around said lever and rigidly connected to said semi-rigid part, and comprising up to four electrically conducting zones, separated by narrow slits, wherein the contacting of each zone with said lever generates the appearance of an electrically conducting path comprising said lever, said zone and said first contact,
- a second contact placed above said lever, so that pressing down said command part on said surface generates a second electrically conducting path comprising said first contact, said second contact and said lever,
- a spring, allowing the interruption of said second electrically conducting path, as soon as said pressure is released.

The invention is equally related to the same device, further comprising a third and fourth contact, being brought into contact with each other by placing the phalanx flat onto the surface, thereby generating an electrically conducting path.

In a particular embodiment of the invention, the activation of said contacting zones is related to movements in the directions left, right, in and out, relative to the user, and wherein activation of said second contact is related to movement in the 'down' direction, relative to the user, and wherein said third and fourth contact are related to the 'up' movement relative to the user.

According to another embodiment of the invention, the movement in at least one direction is commanded by vocal commands, and wherein said second contact allows to enable or disable said vocal command of a movement in said at least one direction.

The invention is equally related to a device as described above, further comprising a support element, for example the handle of a laprascopic instrument, and wherein said command part is a joystick, incorporated into said support element, so that the top of said joystick is accessible to said first phalanx, and wherein said joystick comprises an actuator, preferably a button.

According to another embodiment, said support element comprises an actuator, preferably a button.

According to still another embodiment, joystick comprises a hook-like extension, wherein the first phalanx of the user's finger fits.

In the embodiments comprising a joystick, the device according to the invention may further comprise :
- a central pole, whose vertical movement is spring-operated by way of a spring,
- up to four contacts placed around said pole,
- a first metallic plate connected to said pole,
- a second metallic plate connected to said pole in the lowest point of said pole,
- a contact, activated by pressing down vertically upon said pole.

The device wherein the joystick comprises a hook-like extension may further comprise :
- a contact, activated by pulling out said pole in a vertical direction,
- a proliferation on said support element.

According to another embodiment of a device comprising a joystick, said contact, which is activated by pressing down vertically, enables or disables the vocal command of movement in at least one direction.

### Short description of the drawings

Fig. 1 illustrates the six directions of movement to be controlled by a device according to the invention.

Fig. 2a and 2b two views of a preferred embodiment of the device according to the present invention.

Fig. 3a and 3b illustrate a detailed view of the preferred embodiment of a device according to the present invention.

Fig. 4 illustrates another view of the preferred embodiment of the present invention.

Fig. 5 illustrates a second embodiment of the present invention.

Fig. 6 illustrates a third embodiment of the present invention.

Fig. 7 illustrates a fourth embodiment of the present invention.

Fig. 8 illustrates the operation of a device according to the fourth embodiment of the present invention.

### Detailed description of the invention

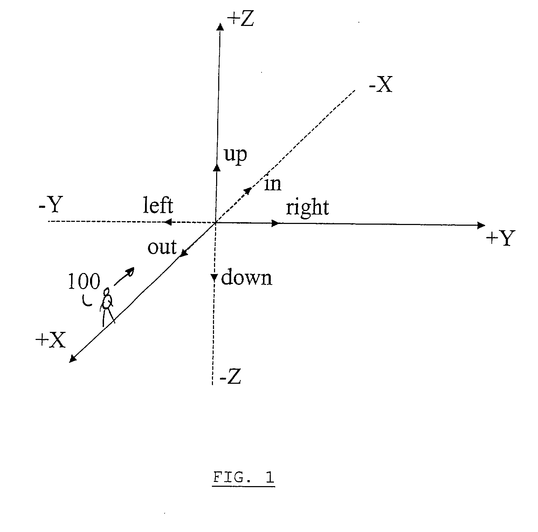

The present invention is related to a device allowing the control of a three-dimensional movement by the user of such a device. With a device according to the invention, movement along three axes may be commanded by movements of the user's finger, the characteristic in all embodiments being that the device is in physical contact only with the first phalanx of one finger. Any one of the five fingers may be used for such a control. In particular, the movement is controlled in a maximum of six directions, being either the positive or the negative of one of three orthogonal axes X, Y, Z (figure 1). In many applications, these directions are more conveniently named left-right/up-down/in-out, depending on the viewpoint of the operator 100 of the device according to the invention, see also figure 1. According to a special embodiment, the movement in a number of these directions may be controlled by other means, in particular by voice control.

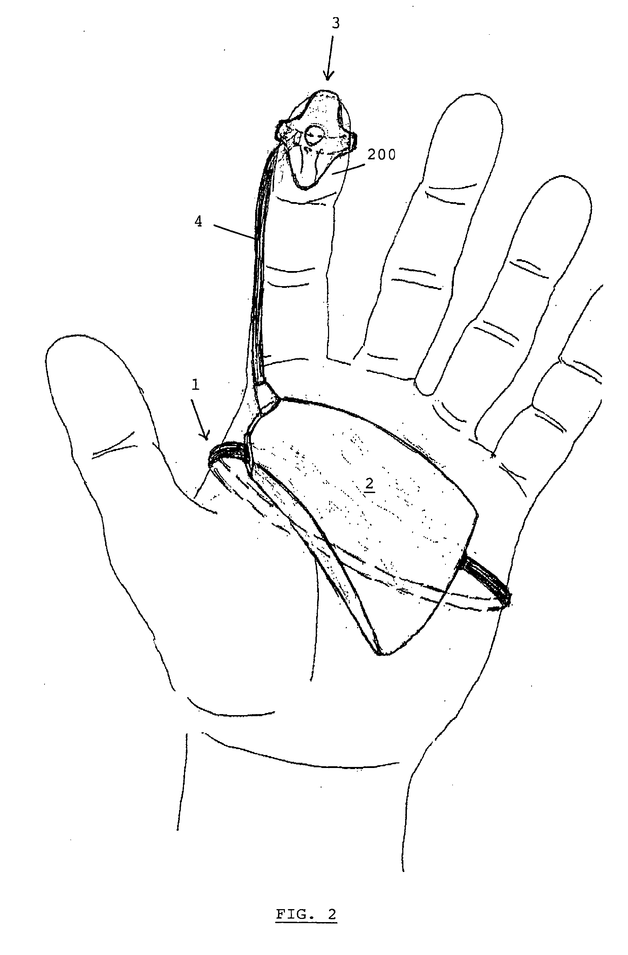

Figure 2 shows a first preferred embodiment of the invention. The device 1 is strapped around the user's hand. A central part 2 is covering the wearer's palm, while the command part 3 is attached to the user's finger, preferably the index finger. The command part 3 is more particularly strapped around the first phalanx 200 of the user's finger. Electrical connections 4 are present between the command part 3 and the central part 2, the latter containing means for sending command signals by wireless transmission to a tool or device such as a laprascopic instrument. Such command signals are then directly related to the signals originating in the command part 3.

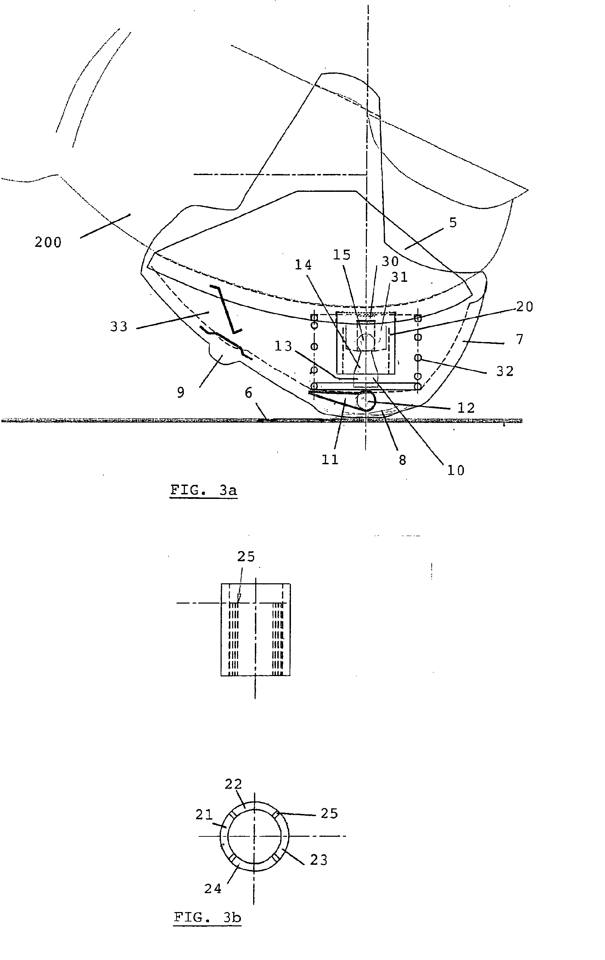

Figure 3a shows a detailed cross sectioned view of this command part 3. First of all, the command part consists of a semi-rigid sub-part 5, that fits on the user's finger's first phalanx 200, as shown in the drawing. Enveloping the finger and making contact with a hard surface 6, is a flexible sub-part 7, comprising on its downside surface two bulging areas 8 and 9. The device is actuated by placing it against the hard surface 6 and making spatial movements with the finger, while maintaining contact with the surface 6 in the same point. In the position shown in figure 3a, movement in five directions can be actuated: up-down, left-right and in. The material of the flexible sub-part 7 is such that considerably friction exists between this sub-part 7 and the rigid surface 6, this way allowing spatial movements of the semi-rigid sub-part 5 relative to the flexible sub-part 7, for example when the finger is pushed forward. During such movements, the contact between part 7 and the surface 6 should be retained in the same contact point.

The first four movements are actuated with the help of a small electrically conducting lever 10, in connection with a first contact 11. The lever comprises a base part 12 which is ball-shaped and which is embedded in the flexible sub-part 7, and making permanent contact with the first contact 11. On top of that, a straight portion 13, a cone shaped portion 14 and a top portion 15 are present, the latter being equally ball-shaped. The lever 10 is installed inside a cylinder-shaped enclosure 20, enabling the second contact. This enclosure 20 is placed underneath and fixed to the rigid part 5 on which the user's finger rests. Figure 3b shows a frontal and plan cross sectioned view of the enclosure 20, which is made up of four electrically conducting zones 21,22,23,24, separated by narrow slits 25.

Each of the four zones is dedicated to the command of movement in one of four directions, preferably left-right and in-out, and four wires (not shown) are connected to these zones, allowing the transmittal of a signal which is relative to a movement in each of these directions to the transmitting device inside the central support 2. The contact is made by the user's movements : by moving his finger from back to front for example, while maintaining contact at the same point of the rigid surface 6, he will cause the enclosure 20, which is fixed to the rigid part 5, to tilt in the forward direction compared to the position shown in figure 3a. The lever, being balanced on its base portion 12, and connected in no rigid way to the semi-rigid part 5, will be induced to tilt in the opposite direction of the enclosure's movement and the two (enclosure and lever) will make contact at the back of the enclosure, more particularly on zone 21 of the enclosure, supposing that the enclosure's orientation in figures 3a and 3b is the same. This contact, along with the base contact, causes an electrical circuit to close, leading to the transmittal of a signal towards the transmitting device inside the support 2 and eventually towards the driven tool. According to the preferred embodiment, a forward or backward movement of the finger will be translated into a movement of the tool in a plane parallel to the XY-plane and in a direction parallel to the X-axis (in or out) of figure 1; a movement in the left or right direction will result in the tool's movement in the left or right direction in said plane parallel to the XY-plane and in a direction parallel to the Y-axis of figure 1.

The narrow slits of the enclosure 20 are such that a simultaneous contact between the lever 10 and two adjacent zones is possible. Provision is made for this type of contact to be translated in a simultaneous movement of the tool in two directions.

In the position shown in figure 3a, a fifth movement ('down', see figure 1) may be actuated. This is done by the third contact 30 placed on the inside of a cylinder-like structure 31 enveloping the top ball shaped part 15 of the lever 10. The structure 31 is flexible to maintain a default position of the lever when the contact 30 is released. The structure 31 acts as a guide for the ball-shaped top part 15 of the lever 10, towards the contact 30, while still allowing a tilting movement of said lever around its base portion 12. The contact 30 is activated by the user's finger pushing down on the command device, thereby causing an electrical contact between the lever's top portion 15 and the contact 30. The 'down' movement resulting from this contact may be in combination with a movement in one or two of the other directions, if the contact 30 is actuated from one of the tilted positions. A spring 32 necessitates the exertion of a minimum pressure by the user, before the contact 30 is actuated. The spring also allows a quick release of the contact 30 once the user's pressure is removed.

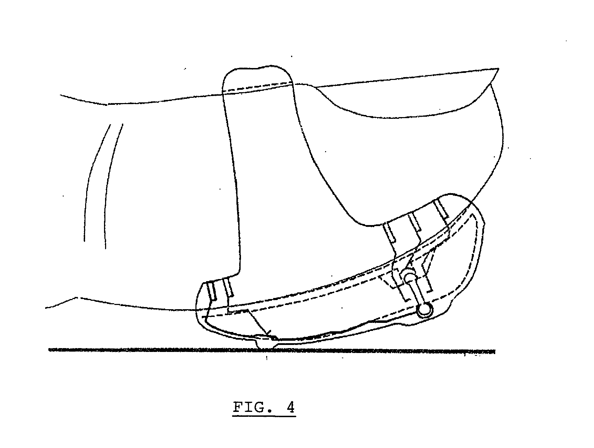

Another pair of contacts 33 is present in the command part, just above the second bulging area 9. By moving the first phalanx 200 into the position shown in figure 4, these contacts 33 are activated. Enabling the contacts 33 in this way activates the sixth movement, namely in the 'out' direction. During the activation of the 'out' movement, all other movements are de-activated.

This device allows a control of the movement in six directions in-out, up-down, left-right, by small movements of one finger. Five directions are controlled by minimal movements of the finger tip, allowing a very accurate control of the object's movements in space. The sixth direction requires a mere tilting of the first phalanx. The user's hand is in no way hampered during operation; a simple lifting of the finger is enough to interrupt the ongoing movements without any delay and thus without errors. The device is very compact and allows a completely wireless operation, thus obviating the necessity of being attached to a device which is to be controlled. An embodiment derived from the one shown in figure 3 controls less than the six directions described. This may be obtained by having less than four conducting zones on the enclosure 20, for example two zones : one contacted by a forward movement, one by a backward movement. The contact 33 may also be left out. A device like that might be used to control a two-dimensional movement, or even a one-dimensional movement.

The contact 11 is preferably made of gold-plated inox. The contacts of the switch 30 are preferably made of gold-plated bronze. The lever is preferably made of gold-plated messing. The spring 32 preferably has four turns, an external diameter between 6.5mm and 7mm, and a diameter of the turns of 0.4mm. According to a preferred embodiment, the height of the envelope 20 is 4.8mm, while the height of the narrow slits 25 is 3.8mm. The width of the slits 25 is preferably between 0.2mm and 0.3mm. Each slit is preferably at a right angle to the adjacent one. The scale of the device may be as much as halved compared to the dimensions described above.

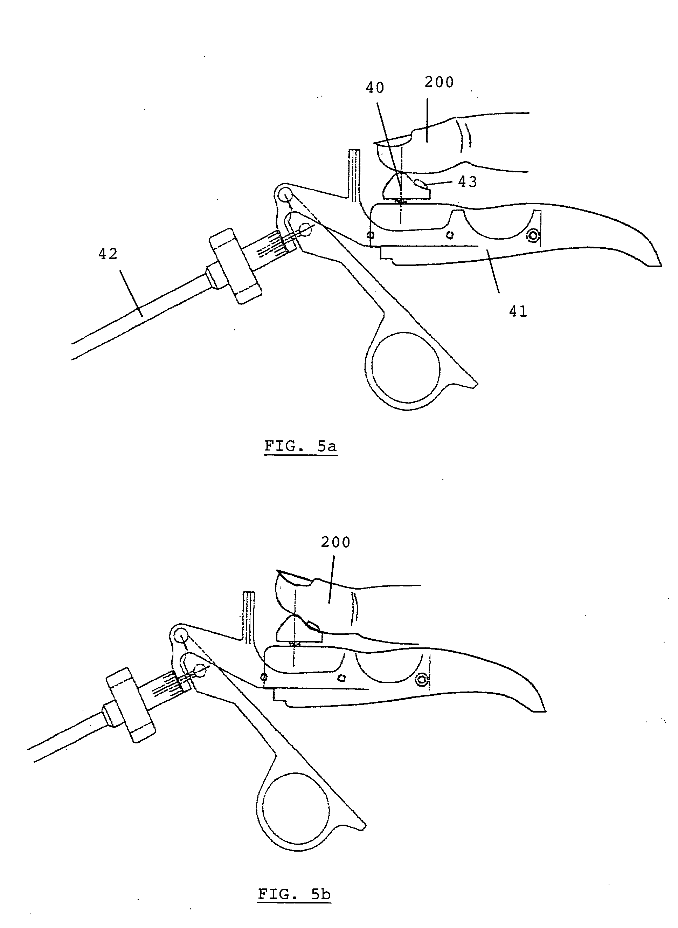

According to a second embodiment of the invention, a joystick 40 is provided, as shown in figure 5. In the embodiment of figure 5, the joystick is incorporated into the handle 41 of an instrument used for laprascopic surgery. Means are provided to send the signals related to movements of the joystick to the driven tool, by wireless transmission. Other embodiments are possible, wherein the joystick is incorporated into a separate device, not connected to the instrument itself. In the embodiment of figure 5, the joystick 40 is moved about by the user's thumb, and this movement is translated into a corresponding movement of a tool (not shown) connected to the rod 42. During all operations, only the first phalanx of the user's thumb is in contact with the joystick 40. The same joystick placed on a flat surface may allow the command of such a joystick by any other finger. In the position shown in figure 5a, five movements are accessible, for example left-right, in-out and down. The movements left-right and in-out are actuated by moving the joystick around with the thumb in the appropriate directions, similar to the movements in the embodiment of fig. 3a. The 'down' movement is actuated by pressing down on the joystick which is equipped with a spring, so that a quick release of the joystick and interruption of the 'down' movement is possible.

The button 43 has the same function as the contact 33 of fig. 3a. By shifting the thumb to the position of figure 5b, thereby pushing the button 43, the OUT-movement is activated. Releasing the button, deactivates said OUT-movement.

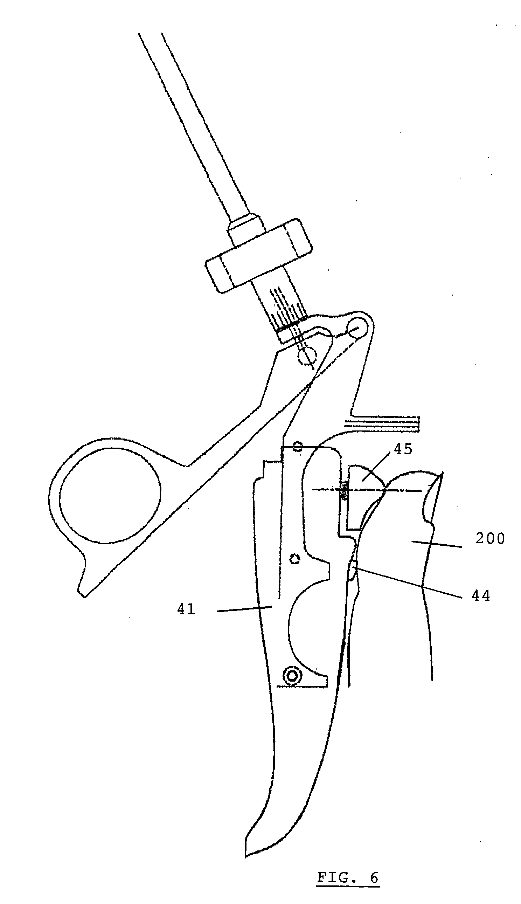

Figure 6 shows an alternative version of the previous embodiment, wherein the button 44 used to command the OUT-movement is placed not on the joystick 45 itself, but on the handle 41 of the instrument.

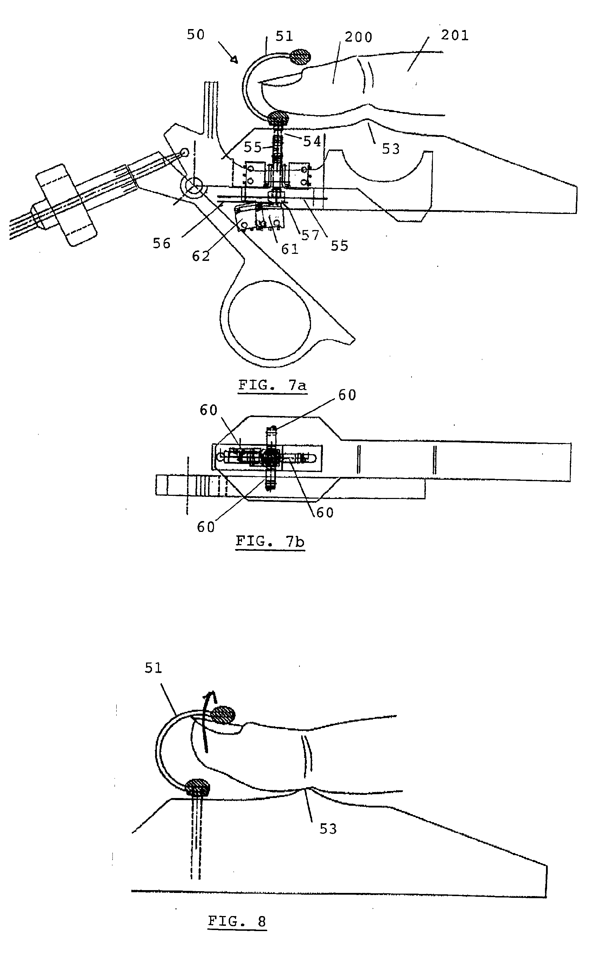

Instead of using a button 43 or 44, the joystick of the invention may be produced according to the embodiment of figure 7, which also shows a laprascopic surgery device equipped with a joystick of the invention. This joystick 50 however has an extension 51 in the form of a hook, allowing the first phalanx 200 of the finger, in this case once more the thumb, to be inserted between this hook and the actual joystick itself. Enlarged portions 52 allow a better contact between the joystick and the thumb. The surface on which this type of joystick is placed comprises a proliferation 53 underneath the joint between the first (200) and the second (201) phalanx of the commanding finger. The control of in-out, left-right and 'down' is then performed in exactly the same way as in the two previous embodiments, but now the command of the 'up' movement is done by pulling out the joystick in the direction of the arrow in figure 8. An upward movement of the first phalanx 200 is helped by resting the finger on the proliferation 53. This activates a contact which in turn activates the 'up' movement.

Technical details of a preferred version of this embodiment are disclosed in figure 7a and 7b (frontal and plan view). The hook is fixed to a supporting pole 54, whose vertical movement is spring-operated by a spring 55. This means that the downward movement of the pole is operated by compressing said spring. After release of a downward force exerted by the finger, the spring restores the pole to a default position. In the vicinity of its lower end point, the pole is fixed to a first metallic plate 55, preferably by a screw and bolt connection, while the extreme low point of the pole rests on a second metallic plate 56, underneath and essentially parallel to the first plate 55. Said low point is preferably constructed in such a way that it has a sphere-like surface 57, allowing the pole to pivot around the contact zone (theoretically a point) between said second plate 56 and said sphere-like surface 57. Meanwhile, the sphere-like surface 57 and the lower plate 56 do need to be connected in a fixed way in their contact point. The pivoting movement will not deform the second plate 56, because of the single contact point, but will deform the first plate 55, which than acts as a spring. Said pivotal movements of the pole 54 are actuated by the movements of the user's finger, and the plate 55 will bring the pole back to its central position as soon as the pole is released.

Around the pole 54, 4 contacts 60 are placed, each enabling command of movement in one of four directions, for example left-right and in-out. These contacts may be contacted by the pole during said pivotal movements, closing an electrical circuit and thereby causing a signal to be sent. Simultaneous actuation of two adjacent contacts 60 may be provided as a possibility, leading to a simultaneous movement in two directions.

Two more contacts are placed underneath the device. The first 61 actuates the fifth movement (for example 'down'), and is activated by the pole being pressed down vertically. The second 62 actuates the sixth movement and is activated by pulling the pole upwards in vertical direction, by way of the hook. This pulling up then bends the second plate 56 upwards, thereby releasing the contact 62. It should be noted that in this set-up, the contact 62 works necessarily in inversion, i.e. movement in the sixth direction is actuated when the contact is released, and interrupted when the contact is re-activated. Naturally, electrical connections (not shown) are present between all contacts and a transmitting device (not shown). Other ways of enabling electrical signals by movements or by pressure of the finger and by the devices shown in figures 2 to 8 may be devised without leaving the scope of the invention.

The devices according to the figures 5 and 6 may operate in much the same way as explained in the last three paragraphs, except that the contact 62 is not present here. The button (43 or 44) is then simply connected to an additional electrical contact, activated by pushing said button.

All of the devices described above are operated by one finger, while the command part is in contact only with said finger's first phalanx 200. More particularly, movement in six directions is commanded by said one finger. The invention is however also related to variants of the above described devices, wherein a number of movements are commanded by other means. These means preferably consist of a vocal command. Known techniques for translating vocal commands into command signals may be used for this. In all devices which have this capability however, an on/off switch has to be present, commanding the activation or the interruption of the vocal command itself. This introduces a security element in the device's operation : for example by pressing a button, the vocal command of a number of movements is enabled, and by releasing said button, this vocal command is disabled.

As an example of such a device, reference is made once more to figure 3. The invention is then related to the same device, without the contact 33, and wherein the contact 30 acts as the control of the vocal command. This vocal command may be able to control the up-down movement, for example, while the left-right and in-out movement are still being commanded by the finger movement, as in the previous embodiment. Pushing down on the device, so that contact 30 is activated, allows 'up-down' to be controlled vocally. Releasing contact 30 blocks the vocal command.

Similar embodiments involving vocal control of a number of movements are to be devised by a person skilled in the art, based on the devices of figures 5 to 8.

## Claims

1. A device for translating spatial movements of a finger of the user of said device, into spatial movements of an object in wired or wireless connection with said device, said device comprising a command part (3) which is connected by electrical connections (4) to a transmission part (2), **characterised in that** said command part is in physical contact only with the first phalanx (200) of said finger, during operation of said device.

2. A device according to claim 1, wherein said movements take place in a maximum of six directions (+X, -X, +Y, -Y, +Z, -Z), each direction being parallel to one of three orthogonal axes (X, Y, Z).

3. A device according to claim 1 or 2, wherein said command part (3) is strapped around said phalanx (200), and held by said phalanx against an essentially rigid surface (6), during operation of said device.

4. A device according to claim 3, wherein said command part (3) comprises :
- a semi-rigid part (5) that fits on the user's finger's first phalanx (200),
- a flexible part (7) enveloping both said semi-rigid part (5) and said phalanx (200) and making contact with said surface (6),
- a first contact (11),
- an electrically conducting lever (10), in permanent contact with said first contact (11),
- a cylinder-like enclosure (20) placed around said lever (10) and rigidly connected to said semi-rigid part (5), and comprising up to four electrically conducting zones (21,22,23,24), separated by narrow slits (25), wherein the contacting of each zone with said lever (10) generates the appearance of an electrically conducting path comprising said lever (10), said zone and said first contact (11),
- a second contact (30) placed above said lever, so that pressing down said command part (3) on said surface (6) generates a second electrically conducting path comprising said first contact (11), said second contact (30) and said lever (10),
- a spring (32), allowing the interruption of said second electrically conducting path, as soon as said pressure is released.

5. A device according to claim 4, further comprising a third and fourth contact (33), being brought into contact with each other by placing the phalanx (200) flat onto the surface (6), thereby generating an electrically conducting path.

6. A device according to claims 4 or 5, wherein activation of said contacting zones (21, 22, 23, 24) is related to movements in the directions left, right, in and out, relative to the user, and wherein activation of said second contact (30) is related to movement in the 'down' direction, relative to the user, and wherein said third and fourth contact (33) are related to the 'up' movement relative to the user.

7. A device according to claims 4 or 5 wherein the movement in at least one direction is commanded by vocal commands, and wherein said second contact (30) allows to enable or disable said vocal command of a movement in said at least one direction.

8. A device according to claim 1 or 2, further comprising a support element (41), for example the handle of a laprascopic instrument, and wherein said command part is a joystick (40), incorporated into said support element, so that the top of said joystick is accessible to said first phalanx (200), and wherein said joystick comprises an actuator, preferably a button (43).

9. A device according to claim 1 or 2, further comprising a support element (41), for example the handle of a laprascopic instrument, and wherein said command part is a joystick (45), incorporated into said support element, so that the top of said joystick is accessible to said first phalanx (200), and wherein said support element (41) comprises an actuator, preferably a button (44).

10. A device according to claim 1 or 2, further comprising a support element (41), for example the handle of a laprascopic instrument, and wherein said command part is a joystick (50), incorporated into said support element, so that the top of said joystick is accessible to said first phalanx (200), and wherein said joystick comprises a hook-like extension (51), wherein the first phalanx (200) of the user's finger fits.

11. A device according to any one of claims 8 to 10, further comprising :
- a central pole (54), whose vertical movement is spring-operated by way of a spring (55),
- up to four contacts (60) placed around said pole (54),
- a first metallic plate (55) connected to said pole (54),
- a second metallic plate (56) connected to said pole (54) in the lowest point of said pole (54),
- a contact (61), activated by pressing down vertically upon said pole (54).

12. A device according to claim 10 or 11, further comprising :
- a contact (62), activated by pulling out said pole (54) in a vertical direction,
- a proliferation (53) on said support element.

13. A device according to claim 11 or 12, wherein said contact (61), which is activated by pressing down vertically, enables or disables the vocal command of movement in at least one direction.

## Amended claims

### Amended claims in accordance with Rule 86(2) EPC.

**1.** A device for translating spatial movements of a finger of the user of said device, into spatial movements of an object in wired or wireless connection with said device, said movements taking place in a maximum of six directions (+X, -X, +Y, -Y, +Z, -Z), each direction being parallel to one of three orthogonal axes (X, Y, Z),.said device comprising a command part (3) which is connected by electrical connections (4) to a transmission part (2), said command part being in physical contact only with the first phalanx (200) of said finger, during operation of said device, **characterized in that** said command part (3) is strapped around said phalanx (200), and comprises means (5,7) which allow said command part (3) to be held by said phalanx against an essentially rigid surface (6), during operation of said device.

**2.** A device according to claim 1, wherein said command part (3) comprises :
- a semi-rigid part (5) that fits on the user's finger's first phalanx (200),
- a flexible part (7) enveloping both said semi-rigid part (5) and said phalanx (200) and making contact with said surface (6),
- a first contact (11),
- an electrically conducting lever (10), in permanent contact with said first contact (11),
- a cylinder-like enclosure (20) placed around said lever (10) and rigidly connected to said semi-rigid part (5), and comprising up to four electrically conducting zones (21,22,23,24), separated by narrow slits (25), wherein the contacting of each zone with said lever (10) generates the appearance of an electrically conducting path comprising said lever (10), said zone and said first contact (11),
- a second contact (30) placed above said lever, so that pressing down said command part (3) on said surface (6) generates a second electrically conducting path comprising said first contact (11), said second contact (30) and said lever (10),
- a spring (32), allowing the interruption of said second electrically conducting path, as soon as said pressure is released.

**3.** A device according to claim 2, further comprising a third and fourth contact (33), being brought into contact with each other by placing the phalanx (200) flat onto the surface (6), thereby generating an electrically conducting path.

**4.** A device according to claims 2 or 3, wherein activation of said contacting zones (21, 22, 23, 24) is related to movements in the directions left, right, in and out, relative to the user, and wherein activation of said second contact (30) is related to movement in the 'down' direction, relative to the user, and wherein said third and fourth contact (33) are related to the 'up' movement relative to the user.

**5.** A device according to claims 2 or 3 wherein the movement in at least one direction is commanded by vocal commands, and wherein said second contact (30) allows to enable or disable said vocal command of a movement in said at least one direction.
